# EUROPEAN PATENT APPLICATION

(11) **EP 3 937 170 A1**
(43) Date of publication of application: **12.01.2022**
(21) Application number: 20185364.5
(22) Date of filing: 10.07.2020
(51) Int. Cl.: G10L 25/66, A61B 5/00, G10L 25/30

(54) **SPEECH ANALYSIS FOR MONITORING OR DIAGNOSIS OF A HEALTH CONDITION**

(71) Applicant: Novoic Ltd., London N1 7GX (GB)
(72) Inventor: WESTON, Jack, London, N1 2LA (GB); FRISTED, Emil, London, N1 8JT (GB)
(74) Representative: Gill Jennings & Every LLP

(57) **Abstract**

The invention relates to a computer-implemented method of training a machine learning model for performing speech analysis for monitoring or diagnosis of a health condition. The method uses training data comprising audio speech data and comprises obtaining one or more linguistic representations that each encode a sub-word, word, or multiple word sequence, of the audio speech data; obtaining one or more audio representations that each encode audio content of a segment of the audio speech data; combining the linguistic representations and audio representations into an input sequence comprising: linguistic representations of a sequence of one or more words or sub-words of the audio speech data; and audio representations of segments of the audio speech data, where the segments together contain the sequence of the one or more words or sub-words. The method further includes training a machine learning model using unsupervised learning to map the input sequence to a target output to learn combined audio-linguistic representations of the audio speech data for use in speech analysis for monitoring or diagnosis of a health condition.

## Description

### TECHNICAL FIELD

The present invention relates to a method and system for training a machine learning model for performing speech analysis, in particular for monitoring or diagnosis of a health condition. The invention also relates to a method of applying the trained machine learning model to patient speech data for monitoring or diagnosis of a health condition and a system incorporating the trained model.

### BACKGROUND

The rapid pace of development in the field of machine learning, together with increasing computing power and the availability of large clinical data sets, has led to the increasing application of computational methods in the analysis, interpretation, and comprehension of medical and healthcare data. The potential for machine learning to transform the healthcare industry is widely recognised and is increasingly looked to as a potential solution to increasing pressures faced in healthcare due to growing ageing populations.

Applications of artificial intelligence in healthcare include the use of machine learning to predict the pharmaceutical properties of molecular compounds and targets for drug discovery; pattern recognition and segmentations techniques on medical images to enable faster diagnosis and tracking of disease progression; and developing deep-learning techniques on multimodal data sources such as combining genomic and clinical data to detect new predictive models.

More recent developments have attempted to analyse spoken language to extract clinically meaningful information. This has involved both text or character based analysis of the linguistic component of speech (e.g. semantics, grammar, syntax, conversational analysis), and audio based analysis of the acoustic components of speech (e.g. prosody, wave-level abnormalities associated with vocal cord functioning). Generally disparate methodologies has been required for analysing these two components. Partly driven by developments in audio processing and natural language processing techniques such as automatic speech recognition, machine learning algorithms have been applied to identify acoustic and linguistic impairments in spoken language indicative of neurodegenerative disorders such as Alzheimer's Disease. Generally these approaches have attempted to extract discriminative features of a patient's speech associated with the use of language, such as repetition or inappropriate use of words, and/or acoustic features such as hesitation and articulation. Generally classification models are then trained to classify data on the basis of the extracted features to diagnose the condition. Despite significant progress, there remains a number of issues with known techniques in the application of machine learning to speech analysis for diagnosis.

Firstly, the diagnosis accuracy of these methods remains limited, partly because existing methods fail to successfully utilise the full extent of the information available in speech data. The algorithms are also often very narrowly focussed, limiting the possibility of applying a given model to other clinical speech analysis applications outside of a particular limited application. For example, the level at which the linguistic information is represented (e.g. word or multiple word level) respectively provides better representation of different information (e.g. semantic and syntactic information). For audio information, representation on different time scales (e.g. relatively longer such as 1.0s or relatively shorter such as 10ms) provides better representation of different information (prosodic information or wave-level information relevant for measuring motor function). Existing methods have had limited applicability working only with certain levels of information.

Another key limitation for medical applications of speech analysis is found in or caused by automatic speech recognition systems (ASR); these systems might introduce noise in two ways, firstly by making errors in detecting correct and/or correctly articulated speech, and secondly in correcting incorrect and/or incorrectly articulated speech (e.g. using a language model). This confounds clinically meaningful information found at the subword level (e.g. mispronunciation, grammatical errors, slurring).

Another key limitation for health applications of speech analysis is the limited availability and size of health related datasets, particularly labelled such datasets. This has limited the application of more expressive models and the generalisability of existing models; as one example of the latter, prior models have generally been limited to working on speech inputs with specific constraints (e.g. someone describing a picture), limiting real world applicability. Other examples where lack of generalisability is still limiting health applications of speech processing is generalising across recording environments, different languages/accents, age, gender, and other nuisance covariates.

Accordingly there exists a need for a method of training a machine learning model for speech analysis tasks that allow the model to extract more clinically relevant information from the speech data to more accurately monitor or diagnose a clinical condition. There is also a related aim to provide a more flexible approach which may be readily applied to different medical conditions, and with generalisability across other domains/nuisance covariates, allowing more widespread application of the technique. Ideally this method should further address the limited availability of clinical datasets.

### SUMMARY OF THE INVENTION

In a first aspect of the invention there is provided a computer-implemented method of training a machine learning model for performing speech analysis for monitoring or diagnosis of a health condition, the method using training data comprising audio speech data, the method comprising: obtaining one or more linguistic representations that each encode a sub-word, word, or multiple word sequence, of the audio speech data; obtaining one or more audio representations that each encode audio content of a segment of the audio speech data; combining the linguistic representations and audio representations into an input sequence comprising: linguistic representations of a sequence of one or more words or sub-words of the audio speech data; and audio representations of segments of the audio speech data, where the segments together contain the sequence of the one or more words or sub-words; the method further comprising: training a machine learning model using unsupervised learning to map the input sequence to a target output to learn combined audio-linguistic representations of the audio speech data for use in speech analysis for monitoring or diagnosis of a health condition.

The present invention also encompasses, in a second related aspect, a computer-implemented method of training a machine learning model for performing speech analysis for monitoring or diagnosis of a health condition, the method using training data comprising audio speech data, the method comprising: obtaining one or more linguistic representations that each encode a sub-word, word, or multiple word sequence, of the audio speech data; obtaining one or more audio representations that each encode audio content of a segment of the audio speech data; forming a linguistic input sequence comprising linguistic representations of a sequence of one or more words or sub-words of the audio speech data; forming an audio input sequence comprising audio representations of segments of the audio speech data, where the segments together contain the sequence of the one or more words or sub-words; the method further comprising: training a machine learning model using unsupervised learning to map the linguistic input sequence and audio sequence to a target output to learn combined audio-linguistic representations of the audio speech data for use in speech analysis for monitoring or diagnosis of a health condition.

The following details are equally relevant to both related methods of the first and second aspect above and may be equally applied to both.

By combining linguistic information associated with the words used in the speech data with non-linguistic information and training the machine learning model on the linguistic and non-linguistic representations jointly, the model is able to learn features associated with the interaction between language and audio components of speech (in addition to features relating solely to language and features relating solely to audio) which provide the model with discriminative abilities not present in existing techniques. In particular, by training the model on an input sequence of linguistic and audio representations the model learns joint audio-linguistic representations capturing information on the interrelation between the language used by a patient and the way it is spoken, including emotion, phonetic errors, deviations and hesitations.

An important insight underlying the invention is that the mutual predictiveness of acoustic, including the prosodic, components and words in speech makes unsupervised training on these inputs a particularly effective means of making audio-linguistic representations.

It is these combined audio-linguistic features learned during training which allow the model to monitor and diagnose clinical conditions with an accuracy not possible with techniques using linguistic and acoustic features without combining them in this way. The model further provides a framework that can be readily trained for application to any of the multitude of clinical conditions in which the interrelation between language and speech acoustics is affected, such as Alzheimer's Disease, Parkinson's Disease, and Depression.

This is particularly evident in the generalisability of the framework across different levels of audio and linguistic information. The linguistic information can for example be captured both at a word or a multiple word level, which respectively provides better representation of semantic and syntactic information. The audio information can for example be captured on a relatively longer time scale (e.g. 1.0s) or a relatively shorter time scale (e.g. 10ms), which respectively provides better representation of prosodic information and wave-level information relevant for measuring motor function.

In particular the framework can similarly be used at a subword level, e.g. the phoneme or character level. This addresses a key limitation for medical applications of speech analysis found in or caused by automatic speech recognition systems (ASR); these systems might introduce noise in two ways, firstly by making errors in detecting correctly pronounced words, and secondly in correcting incorrectly pronounced words (e.g. using a language model). This confounds clinically meaningful information found at the subword level (e.g. mispronunciation, grammatical errors, slurring). This invention is particularly well suited for capturing this information: firstly by having the ability to work at the subword level, and secondly by being able to capture information in the interaction of the audio and language.

The invention's use of unsupervised learning during training enables pre-training on large general purpose datasets, before training on smaller task specific datasets. This benefit addresses another key limitation for health applications of speech analysis: the limited availability and size of health related datasets, particularly labelled such datasets.

Preferably a representation comprises a feature vector, i.e. a vector encoding important distinguishing attributes of the input data. The term embedding is used interchangeably with the term representation. Preferably a representation captures meaningful structure of the input by placing meaningfully similar inputs close together in the representation space. A representation can be learned and reused across models or at different stages of training.

A "health condition" is considered to encompass any theoretically measurable property associated with a human, either physical, physiological, psychological, or mental state or trait. Monitoring and diagnosis is considered to cover the prediction, tracking, evaluation, study or any other type of analysis or identification relating to a health condition.

Audio speech data preferably comprises raw audio data containing speech, including sounds vocalised by humans. The input may further comprise text data where preferably the text data comprises a transcript of the audio speech data.

A "target output" preferably comprises output data to which the model is trained to map the audio and linguistic representations to. A target output preferably comprises any target data which requires the model to learn audio-linguistic representations in the process of mapping the input to the output. It may take many different forms, for example one or more of a target output data sequence, a class or category, a value, score or distribution. The output may be a sequence of combined audio-linguistic representations.

The machine learning model preferably comprises any model suitable for mapping sequences of audio and linguistic representations to a combined audio-linguistic representation space. Preferably the machine learning model comprises an encoder for mapping the input sequences of audio and linguistic representations to combined audio-linguistic representations. Preferably the machine learning model preferably additionally comprises a decoder for mapping the combined audio-linguistic representations to the target output. Preferably, in these examples, training comprises training the encoder and decoder together to map the input sequence of audio and linguistic representations to the target output. The decoder may be discarded after training (e.g. after pre-training) and the trained encoder used for downstream speech analysis tasks.

In some examples the machine learning model comprises a sequence-to-sequence model, where the model may comprise a sequence encoder and a sequence decoder, the sequence encoder for mapping the input sequence to combined audio-linguistic representation and the sequence decoder for mapping the combined audio-linguistic representations to a target output sequence. The target output sequence may comprise one of: a sequence of audio and linguistic representations; a sequence of text and/or audio data. The sequence decoder may be discarded after one or more training steps are completed (e.g. after pre-training) and the trained sequence encoder used for further training.

Preferably the model comprises an attention-based model, for example a model that uses contextual information of an input sequence to form context-dependent representations, for example a model that uses the context of an element in an input sequence, where context comprises for example the position and relationship of an element in the input sequence relative to other elements in the input sequence, where an element may refer to a sub-word, word or multiple word segment, an audio segment or a sequence position. The model may comprise a Transformer, for example including a Transformer encoder and Transformer decoder.

Preferably the machine learning model comprises training using self-supervised learning. Preferably self-supervised learning comprises learning on unlabelled data where the model creates labels using properties of the data. Preferably it involves training the model to withhold a part of the input data and training the model to predict the withheld part of the data.

Preferably training the machine learning model comprises masking or corrupting one or more of the linguistic and/or audio representations in an input sequence and training the machine learning model to predict the masked or corrupted linguistic and/or audio representations. Preferably the model comprises a sequence to sequence model and the model is trained to map an input sequence comprising one or more hidden, masked or corrupted representations to an output sequence in which the hidden, masked or corrupted representations are predicted. Preferably the encoder maps the input sequence to an audio-linguistic representation space and the decoder maps the audio-linguistic representations to the output sequence. Preferably the encoder-decoder are trained together such that the encoder learns, based on the interdependence of the audio and linguistic information, combined audio-linguistic representations which are predictive of the masked corrupted or hidden representations.

In some examples the method comprises forming a linguistic sequence comprising linguistic representations of a sequence of one or more words or sub-words of the audio speech data; forming an audio sequence comprising audio representations of segments of the audio speech data, where the segments together contain the sequence of the one or more words or sub-words; and combining the linguistic sequence and audio sequence into an input sequence. The combining may take place before the first layer of the machine learning model or after one or more layers of the machine learning model.

Preferably combining the linguistic representations and audio representations comprises: forming a linguistic sequence comprising linguistic representations of a sequence of one or more words or sub-words of the audio speech data; forming an audio sequence comprising audio representations of segments of the audio speech data, where the segments together contain the sequence of the one or more words or sub-words; and combining the linguistic sequence and audio sequence by one or more of: concatenating the linguistic sequence and audio sequence along any dimension; summing the linguistic sequence and audio sequence; performing a linear or non-linear transformation on one or both of the audio sequence and linguistic sequence; combining the linguistic sequence and audio sequence by inputting to an initial neural network layer.

In some examples combining the linguistic sequence and audio sequence comprises: training a neural network layer to align the audio sequence with the linguistic sequence by, for each linguistic representation, selecting one or more relevant audio representations using temporal alignment information, where the model obtains the temporal alignment information from the audio sequence by determining the time delays between the linguistic representation and each audio representation. This method is advantageous as it allows for initial pre-training of the model on text-only data using conventional techniques before training on audio and text data to learn combined audio-linguistic representations.

Preferably the method further comprises performing a subsequent task-specific training step using task-specific training data associated with a specific health monitoring or diagnosis task. In this way, pre-training can be carried out on large widely available unlabelled general purpose data sets and more limited health related data sets are only required for a subsequent task-specific training step to optimise the model for a particular speech processing task. This allows for significant performance gains despite limited specific data.

Preferably, performing task specific training comprises providing task-specific training data comprising audio speech data, the method comprising: obtaining one or more linguistic representations that each encode a sub-word, word, or multiple word sequence, of the audio speech data; obtaining one or more audio representations that each encode audio content of a segment of the audio speech data; combining the linguistic representations and audio representations into an input sequence comprising: linguistic representations of a sequence of one or more words or sub-words of the audio speech data; and audio representations of segments of the audio speech data, where the segments together contain the sequence of the one or more words or sub-words; the method further comprising: adding a task-specific network layer after the pre-trained machine learning model; training either the task-specific layer alone or the pre-trained machine learning model and task-specific model so that the input sequence is mapped by the pre-trained machine learning model and task-specific model to a target output associated with a task-specific health condition. Training may comprise unsupervised, self-supervised, semi-supervised or supervised learning.

Preferably the method comprises pre-training the machine learning model using unsupervised learning on a first training data set to map the input sequence to a target output to learn combined audio-linguistic representations of the audio speech data; adding a task-specific network layer after the machine learning model and performing task-specific training using a second training data set comprising task-specific training data.

In particular, where the machine learning model comprises an encoder, the encoder may be pre-trained on a first, preferably unlabelled data set, using unsupervised or semi-supervised learning. A task-specific layer is then added to the model where the task-specific layer takes as input the audio-linguistic representations from the pre-trained encoder and performs an operation to map the audio-linguistic representations to a target output related to health condition. Where the machine learning model comprises an encoder-decoder, the method may comprise pre-training the encoder-decoder using unlabelled data using unsupervised or semi-supervised learning to map the input sequence to a pre-training target, for example using a de-noising objective, the method comprising discarding decoder after pre-training and appending the task-specific network layer to the pre-trained encoder.

The task-specific network layer may be one or more of: a classifier, a regression model, a clustering model or a sequence encoder, where the task-specific network layer may be selected based on a chosen speech processing task.

For example the task specific network layer may be one or more of:
- a classifier trained to map the output audio-linguistic representations from the pertained encoder to one or more categories, for example for classification of speech data as falling into a class associated with a particular health condition;
- a regression model trained to provide a numerical value associated with a particular health condition based on the input audio-linguistic representations, for example to give a value associated with a health condition severity score;
- a sequence decoder which decodes the input audio linguistic representations to an output sequence, for example to describe a change in an indicated disease overtime, where the model may be trained on labelled data using supervised training;
- a clustering model which uses unlabelled data and is trained using unsupervised learning to sort the data into clusters with similar properties based on the input audio-linguistic representations, where this clustering of the data may be used to extract previously unknown health related trends in the input speech data.

Task-specific training may comprise: training the pre-trained machine learning model and the task-specific layer together to map an input sequence to a target output associated with a health condition. In this way the pre-trained machine learning model continues to learn refined audio-linguistic representations for a particular health task.

Alternatively task-specific training may comprise fixing the pre-trained machine learning model after pre-training; mapping an input sequence of the task-specific training data to audio-linguistic representations using the pre-trained machine learning model; and training the task-specific network layer to map the audio-linguistic representations to a target output associated with a health condition.

Preferably task specific training comprises training the pre-trained model using unsupervised learning. Preferably in this case the training data is unlabelled data where some of the data may relate to speech data relating to someone with a health condition.

In some examples the task-specific network layer comprises a clustering model and task-specific training comprises training the clustering model to cluster audio-linguistic representations of the task-specific training data learned by the machine learning model according to a health condition. In this way the model can be applied to identify patterns relating to health conditions in unlabelled data, for example to monitor or identify undiagnosed conditions.

In some examples the method comprises training the pre-trained model using supervised or semi-supervised learning using labelled training data where the labels are associated with a health condition. For example, where the task specific network layer comprises a classification model, task-specific training may comprise training the classification model to map audio-linguistic representations of the task-specific training data to a class associated with a health condition.

In some examples the health condition is related to the brain, for example a cognitive or neurodegenerative disease (example: Dementias, Alzheimer's Disease, Mild Cognitive Impairment, Vascular Dementia, Dementia with Lewy Bodies, Aphasias, Frontotemporal Dementias, Huntington's Disease); motor disorders (example: Parkinson's Disease, Progressive Supranuclear Palsy, Multiple System's Atrophy, Spinal Muscular Atrophy, Motor Neuron Disease, Multiple Sclerosis, Essential Tremor); affective disorders (example: Depression, Major Depressive Disorder, Treatment Resistant Depression, Hypomania, Bipolar Disorder, Anxiety, Schizophrenia and schizoaffective conditions, PTSD); neurobehavioural conditions (example: spectrum disorders, Attention-Deficit Hyperactivity Disorder, Obsessive Compulsive Disorder, Autism Spectrum Disorder, Anorexia, Bulimia), head injury or stroke (example: stroke, aphasic stroke, concussion, traumatic brain injury); pain (example: pain, quality of life)

Preferably the health condition is related to one or more of a cognitive or neurodegenerative disease, motor disorder, affective disorder, neurobehavioral condition, head injury or stroke. The methods according to the present invention are able to extract signals relating to the interrelation of language and speech which are particularly affected by changes in the brain and therefore the method is particularly optimised for detecting them.

In some examples the health condition is related to the respiratory system for example: SARS-CoV-2, Whooping cough, Asthma, COPD, Pneumonia, Wet/dry cough, Flu, Common cold, Lower respiratory Infections; Trachea, Bronchus, and Lung cancers; Tuberculosis).

In some examples the health condition comprises multiple health conditions or symptoms of different health conditions, or where the health conditions are not yet known.

Preferably the linguistic representations each encode a character or phoneme of the audio speech data. In this way, the model can learn fine detail in the interaction between the linguistic content and acoustic content of speech. As described above, this addresses a key limitation for medical applications of speech analysis which generally process longer text sequences and introduce noise, for example due to correcting mispronounced character sequences.

Preferably the audio representations comprise prosodic representations that each encode non-linguistic content of a segment of the audio speech data. The term "prosody" used herein is intended to refer to all acoustic information in the input audio speech data except for the normative phonetic information, i.e. the information which is used to transcribe speech.

The prosodic representations preferably encode all acoustic information in the input audio speech data other than the normative phonetic information. To define in a second way, prosody representations preferably encode all acoustic information other than the words used by the speaker. The prosody representations preferably encode acoustic information comprising one or more of, and preferable all of, speaker identity (e.g. gender, age, accent), emotionality, intonation, inflections, acoustic environment.

By using prosodic representations in the input sequence, the model learns joint prosodic-linguistic representations of the audio speech data during training. Since the model uses the prosody of the input speech to help predict the text content of the input speech and vice versa, the model learns strong joint prosodic-linguistic representations. These representations carry rich information on the interaction between prosody and linguistics in the input speech data which can be used to monitor, identify, diagnose or otherwise evaluate a health condition.

Obtaining a prosodic representation may comprise inputting a segment of audio data into a prosody encoder trained to map an audio speech data segment to a prosodic representation encoding non-linguistic content of the audio speech data segment. In particular the prosody encoder may form a fixed part of the model which takes as input an audio data segment or audio representation and provides as output a prosodic representation.

In some examples of the invention the prosody encoder is trained by: training a sequence-to-sequence autoencoder comprising an encoder for mapping input audio data to a reduced dimension representation and a decoder for reconstructing the input audio data from the reduced dimension representation; conditioning the autoencoder by providing information on the linguistic content of the audio data during training such that the autoencoder learns representations which encode the non-linguistic content of the input audio data; using the trained encoder of the autoencoder as the prosody encoder.

In some examples of the invention the prosody encoder is trained by: performing vector quantisation on segments of audio data of 0.9 s or longer. By training the encoder of lengths of audio data of this size, the encoder can be trained to encode just the prosodic information.

Preferably each linguistic representation comprises a text token indicating a subword, word or multi-word sequence from a fixed-size unified vocabulary.

Preferably each audio representation comprises an audio token indicating a vector quantized audio representation encoding the audio content of a segment of audio data containing one or more words or subwords; wherein together the text tokens and audio tokens form a fixed-size audio-linguistic vocabulary, such that any input segment of audio speech data can be represented by a sequence of text tokens and audio tokens. The fixed size audio-linguistic vocabulary may further comprise one or more of: a corruption token, used to mask a text token or audio token for the purposes of self-supervised learning; or a special token, such as a start of sequence, start of linguistic sequence and/or start of audio sequence token, where the special tokens are used to aid the model's processing of the token sequence. In examples using prosodic representations, the audio tokens may be replaced with prosody tokens indicating quantised prosodic representations.

In some examples the training data further comprises one or more further types of sequential data which are temporally associated with the audio speech data wherein the method further comprises: obtaining one or more further data type representations that each encode information of a temporal segment of the further data type; combining further data type representations into the input sequence, where the further data type representations encode data from temporal segments which overlap with the audio representations; training the machine learning model using unsupervised learning to map the input sequence to a target output to learn combined multi-modal representations of the audio speech data and further data type for use in a speech processing task. By extending the training method to train on additional modes which are linked to speech, further relational information can be extracted between the different types of information to further increase the ability of the model to monitor or identify a health condition. All of the details of the model described herein may equally be applied to other additional types of input data which is temporally associated and mutually predictive with the speech data in such a way that the model can learn strong multi-modal representations.

In some examples, separate input sequences are formed for each type of representations and the sequences are combined and the model is trained to map the input sequences to a target output.

The further data type may comprise one or more of: video data comprising one or more of facial expressions, eye movements, actions, gestures, body movements exhibited by a speaker of the audio speech data; external stimuli such as audiovisual prompts; speaker, action or stimulus identifiers.

In a further aspect of the invention there is provided a computer-implemented method of using a machine learning model for performing speech analysis for monitoring or diagnosis of a health condition, the method using user data comprising audio speech data task the method comprising: obtaining one or more linguistic representations that each encode a word or sub-word of the audio speech data; obtaining one or more audio representations that each encode audio content of a segment of the audio speech data; combining the linguistic representations and audio representations into an input sequence comprising: linguistic representations of a sequence of one or more words or sub-words of the audio speech data; and audio representations of segments of the audio speech data, where the segments together contain the sequence of the one or more words or sub-words; the method further comprising: inputting the input sequence of into a machine learning model trained according to the method described above or in any of the appended claims to provide an output associated with a health monitoring or diagnosis task.

By applying the trained model to speech data, the trained model can be used to identify, monitor, diagnose or otherwise evaluate a health condition. Since the training method of the present invention causes the model to learn strong combined audio-linguistic (and potentially other modes) representations, the trained model may be highly predictive of a large range of health conditions.

In some examples the output of the trained model is a sequence of audio-linguistic representations of the user audio speech data, the method may further comprise using the audio-linguistic representations for a downstream speech analysis task. In a further aspect of the invention there is provided a system for performing speech analysis to provide monitoring or indication of a health condition, the system comprising data processing means configured to perform the method of using the trained model as defined above or in any of the appended claims.

In a further aspect of the invention there is provided a medical device for performing speech analysis to provide monitoring or indication of a health condition, the system comprising data processing means configured to perform the method of using the trained model as defined above or in any of the appended claims. The medical device may comprise an input for collecting patient audio speech data, a processing unit for analysing the input patient speech data using the machine learning model trained as described above or in any appended claim, and an output for providing an indication related to monitoring or identifying a health condition.

In a further aspect of the invention there is provided a system for training a machine learning model comprising data processing means configured to perform the training method defined above or in any of the appended claims.

In a further aspect of the invention there is provided a computer-implemented method of training a machine learning model for performing a speech processing task, the method using training data comprising audio speech data, the method comprising: obtaining one or more linguistic representations that each encode a sub-word, word, or multiple word sequence, of the audio speech data; obtaining one or more audio representations that each encode audio content of a segment of the audio speech data; combining the linguistic representations and audio representations into an input sequence comprising: linguistic representations of a sequence of one or more words or sub-words of the audio speech data; and audio representations of segments of the audio speech data, where the segments together contain the sequence of the one or more words or sub-words; the method further comprising: training a machine learning model using unsupervised learning to map the input sequence to a target output to learn combined audio-linguistic representations of the audio speech data for use in a speech processing task.

Although the methods of the present invention are preferably directed to speech analysis for monitoring and diagnosis of a health condition, the methods may equally be applied to more general speech processing tasks. This requires no modification to the methods described herein, other than the choice of the training data and training objective for the task specific training step. Examples of suitable speech processing tasks include but are not limited to: Automatic speech recognition; diarisation (separating speakers during automatic speech recognition); Lie detection; Sarcasm detection Personality prediction; Sentence acceptability; Sentiment analysis; Paraphrasing/sentence similarity; Natural language inference; Coreference resolution; Sentence completion; Word sense disambiguation; Question answering; Machine translation; Understanding intent; Conversational agents such as chatbots.

In particular, pre-training is carried out exactly as described herein with no modifications required. The audio-linguistic representations learned during pre-training are equally applicable to the types of speech processing tasks described above.

The method of analysis for speech processing tasks further preferably comprises performing a subsequent task-specific training step using task-specific training data associated with a specific speech processing task. In particular, it preferably comprises: pre-training the machine learning model using unsupervised learning on a first training data set to map the input sequence to a target output to learn combined audio-linguistic representations of the audio speech data; adding a task-specific network layer after the machine learning model and performing task-specific training using a second training data set comprising speech processing task-specific training data. The method is exactly the same as described throughout, other than the task-specific network layer is trained to perform a speech processing task, rather than a health related task.

### BRIEF DESCRIPTION OF THE DRAWINGS

Embodiments of the invention will now be described, by way of example only, with reference to the accompanying drawings, in which:
Figure 1 schematically illustrates an overview of a method of pre-training a machine learning model according to the present invention;
Figure 2 schematically illustrates an overview of a method of performing task-specific training of a machine learning model according to the present invention;
Figure 3 schematically illustrates a specific example of a method of pre-training a machine learning model according to the present invention;
Figure 4 schematically illustrates a specific example of a method of performing task-specific training of a machine learning model according to the present invention;
Figure 5 schematically illustrates a specific example of a method of pre-training a machine learning model according to the present invention;
Figure 6 schematically illustrates a specific example of a method of performing task-specific training of a machine learning model according to the present invention;
Figure 7 schematically illustrates a method of training a prosody encoder for use in the method according to the present invention;
Figure 8 illustrates a specific example of a method of training a prosody encoder for use in the method according to the present invention;
Figure 9 illustrates a training sequence for training a machine learning model according to the present invention.

### SPECIFIC DESCRIPTION

Speech production is regulated by the interaction of a number of different physical, physiological, and psychological systems in the human body. At the higher levels it requires use of a number of different areas of the brain including those for memory to recall thoughts and concepts, the brain areas for sentence construction and word-recall in order to form the concepts into sentences, and then represented as syllables and phonemes; and the brain areas that form phonetic representations to position and control of the vocal cord and other articulators of the articulatory system to control these organs to produce the required sounds for syllables and phonemes. Speech production is also dependent on these parts of the body themselves, including a healthy and correctly functioning vocal cord for correct positioning of the articulators and vocal folds, correct functioning of the articulatory system including timing and coordination of the articulators and vocal folds, a healthy and correctly functioning respiratory system for producing the airflow that's is converted into speech-and neural signalling that controls these system, for example for muscle activation.

There are a large range of diseases that impinge upon the correct functioning of these physiological systems. Cognitive disorders such as Alzheimer's affect the brain and therefore impact on speech through both the higher-level speech systems such as memory but also the lower-level physiology in terms of the brain's ability to control the vocal cord and articulatory system. Affective disorders such as depression result in different types of changes. Choice of language may be affected but also there are observable changes related to prosodies, i.e. non-linguistic effects due to a change in the way the vocal cords are used to produce speech. Motor disorders such as Parkinson's result in changes due to a deterioration in control of the vocal cord and articulatory systems, whereas respiratory disorders such as Pneumonia inhibit the airways and again affect the regular functioning of the articulatory system.

Since different health conditions affect different combinations of components of the overall speech production system, and impact those combinations in unique ways, changes in a person's speech carry signals which can be used to diagnose, monitor and evaluate health-related conditions. Many of these changes are extremely subtle and can arise long before symptoms arise that can be picked up by conventional tests. The possibility of identifying these conditions more effectively and at an earlier stage based on these speech changes ("speech biomarkers") allows for earlier and more effective treatment, and also reduces the significant healthcare costs associated with caring for patients with advanced forms of these conditions.

In order to identify speech biomarkers which can be used to identify these conditions, a model must be trained to extract both linguistic, i.e. language, features and also acoustic speech information. However the inventors of the present application have additionally identified that, given the inter-dependence of the language and acoustic features within speech due to interaction of the physiological systems described above, to obtain the most reliable identification of these conditions, *relational* information between the acoustic and linguistic information must be extracted, in addition to information related to acoustics and language separately.

The present invention centres on this new concept of extracting meaningful combined audio-linguistic representations from speech data and using these combined representations to infer health related information, applicable to a huge range of applications. The invention preferably achieves this by training a machine learning model in two stages. The first uses widely available unlabelled data to learn combined audio-linguistic representations encoding the relational information being sought. The second step involves task-specific training for a particular health monitoring or evaluation application using potentially much smaller clinical data sets to further refine the combined audio-linguistic representations to enhance their predictive and discriminative properties for health applications. It is widely accepted that, for most models, training on larger amounts of data provides greater improvements than architectural optimisation of the model itself, so the present method overcomes the problem of limited clinical data sets by pre-training on large general purpose speech data sets.

### Overview of two-step training process

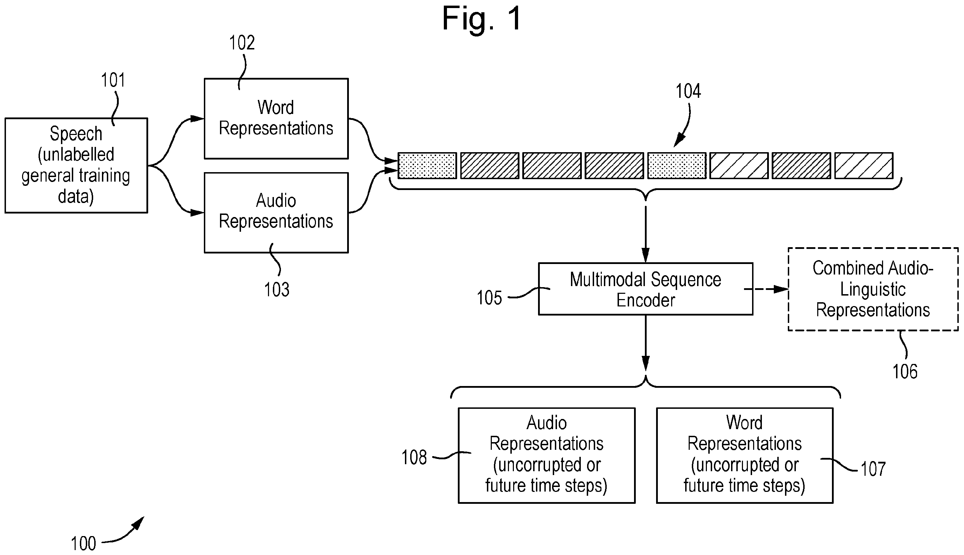

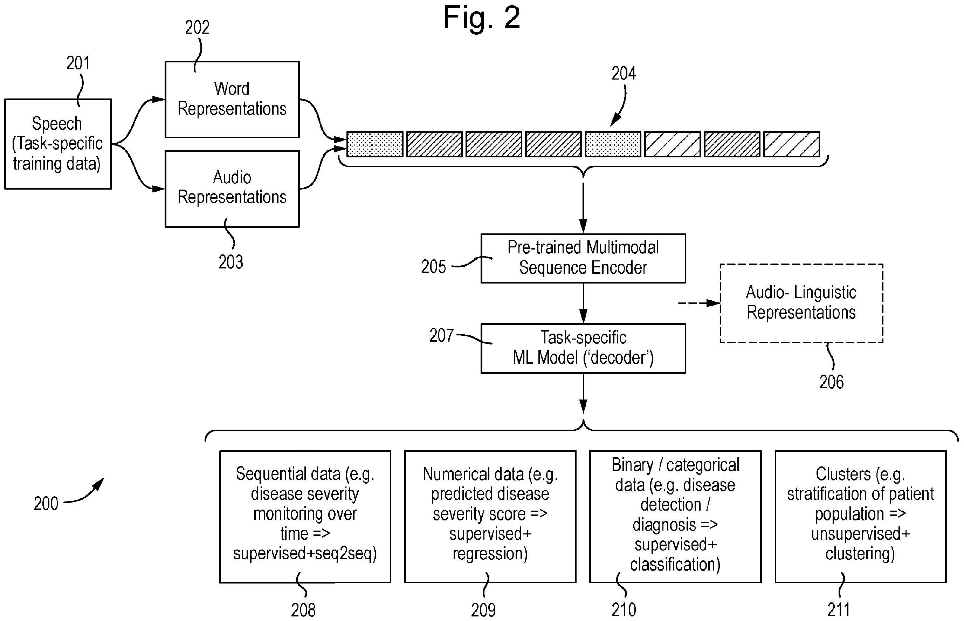

Figures 1 and 2 schematically illustrate an overview of the method of training a machine learning model for performing speech analysis for monitoring or diagnosis of a health condition according to the present invention. In particular, Figure 1 illustrates the "pre-training" method 100, on which a model is trained to learn combined audio-linguistic representations of speech data, using unsupervised learning on large audio speech data sets, and Figure 2 illustrates the secondary "fine-tuning" method 200 in which, after pre-training, task-specific training is carried out using a health-related task-specific training data set, as will be described.

Starting with an overview of the pre-training method 100 illustrated in Figure 1, the method is applied to training data 101 comprising audio speech data. The first step involves obtaining one or more linguistic representations 102 that each encode a sub-word, word or multiple word sequence of the audio speech data 101. As will be explained in more detail below, the words may be extracted from the audio speech data 101 using automatic speech recognition (ASR) or alternatively a transcript may be provided as text data as part of the input training data 101. The method further includes obtaining one or more audio representations 103 that each encode audio content of a segment of the audio speech data 101. The linguistic representations 102 and audio representations 103 are combined into an input sequence 104 where the input sequence comprises linguistic representations of a sequence of one or more words or sub-words of the audio speech data 101 and audio representations of segments of the audio speech data 101, where these segments together contain a sequence of one or more words or sub-words. Explained in a different way, the audio representations and linguistic representations generally correspond to related parts of the audio speech data (or indeed they might be precisely aligned by the model), which is required to allow relational information to be extracted. The method 100 finally comprises training a machine learning model 105 using unsupervised learning to map the input sequence 104 to a target output 107, 108 to learn combined audio-linguistic representations 106 of audio speech data 101 for use in speech processing for monitoring or diagnosis of a health condition.

The model 105 may be any model structured to take an input sequence of representations and map these to a target output 107, 108 which requires an intermediate combined audio-linguistic representation to be learnt during the training. In particular, the model comprises a sequence encoder 105 for mapping the input representations to the combined representations. The model may include a decoder to map the input representations to some kind of interpretable output such as an output sequence of audio representations or word representations.

The model 105 is trained using self-supervised learning (a subset of unsupervised learning) in which the model is trained to predict some property of the input sequence 104. For example, labelled data may be created by the model out of unlabelled data by training the model to predict a masked, corrupted or otherwise hidden parts of the input data by mapping the corrupted data to an uncorrupted version of the data, thereby training the model 105 to learn combined audio-linguistic representations which allow the model to predict the masked or corrupted elements due to the interdependence of the text and audio representations within the input sequence 104.

The model preferably comprises a sequence to sequence model in which an input sequence 104 is mapped via the audio-linguistic representation space to an output sequence. In particular, an input sequence in which one or more of the representations are corrupted or hidden is mapped to an output sequence in which the masked representation is present in the sequence. One particular example of a suitable model is based on the Transformer architecture (see "Attention is all you need", Vaswani 2017, arXiv:1706.03762v5), in which the model 105 includes a Transformer encoder, as described in more detail below. In the following description the term "encoder" is used to describe the part of the model which is trained during pre-training to map the input sequence to audio-linguistic representations, and is carried forward to the fine-tuning stage. The model may have additional parts which are discarded after the pre-training stage.

This type of self-supervised learning techniques, for example using these "denoising" objectives, allow the model to be trained on widely available large unlabelled data sets. By training the model 105 on large amounts of speech data (comprising text and/or audio data) using such self-supervised training methods the model 105 learns audio-linguistic representations encoding information about the relationship between the audio and speech data which isn't present in either of these data types when considered alone. As described above, it is this rich relational information in the audio and text components of speech data which may be used to predict, monitor, diagnose or otherwise evaluate health related information at an accuracy not possible using previous speech analysis techniques utilising the linguistic analysis or audio analysis alone.

Although the combined audio-linguistic representations learnt in the process of figure 1 may be used immediately for clinically relevant applications, preferably the method further involves a fine tuning step, an overview of which is illustrated in Figure 2. In this step, the method involves using task-specific training data comprising audio speech data (and optionally transcript text data) related to a particular task for optimisation. In particular, for training for a health application, health related speech data is used to further train the model, using a task specific target output.

The same approach as described above with Figure 1 is used to prepare the health related training data (e.g. patient speech data where the patient has a particular health condition, and comparable speech data from healthy controls) 201 appropriately to train the model. This involves obtaining word representations 202 and audio representations 203 from the speech data, where again the word representations may be obtained from audio data using known automatic speech recognition algorithms, or alternatively extracted directly from text transcript data provided with the audio speech data. The word representations 202 and audio representations 203 are combined as before into an input sequence 204 which comprises audio and word representations corresponding to a sequence of the clinically relevant speech data 201.

As before, the input sequence must comprise linguistic representations of a sequence of one or more words or sub-words of the input audio speech data and audio representations of segments of the audio speech data which together contain the sequence of the one or more words or sub-words. An important case is character-level linguistic representations, which naturally encode both semantic (word) representations and phonetic information, obviating the need for an external temporal alignment signal and allowing the network to learn the complementary tasks of ASR and TTS through the denoising objective. In this case, the pre-trained model 205, taken after the pre-trained method of Figure 1, is used to map the input sequence 204 to a target output 208, 209, 210, 211. To do this, the trained encoder 205 in Figure 1 is appended with a task specific machine learning model (e.g. comprising a decoder) which takes and inputs the combined audio-linguistic representations of the pre-trained encoder and performs some operation on these to transform them to a target output. The target output may be sequential data (e.g. disease severity monitoring over time) 208, numerical data (e.g. a predicted disease severity score) binary or categorical data where the output audio-linguistic representations are mapped to one or more categorises (e.g. for diseased diagnosis) or clusters (e.g. the stratification of patient population using unsupervised learning to cluster the health related training data to find trends in unlabelled data).

The task-specific machine learning model 207 added to the model 205 will depend on the specific health related task chosen. These applications will be discussed in more detail below but important examples include:
- A classifier trained to map the output audio-linguistic representations from the pretrained encoder 205 to one or more categories, for example for classification of speech data as falling into a class associated with a particular health condition;
- A regression model trained to provide a numerical value associated with a particular health condition based on the input audio-linguistic representations, for example to give a predicted head injury severity score;
- A sequence decoder which decodes the input audio-linguistic representations to an output sequence, for example to describe a change in an indicated disease over time, where the model may be trained on labelled data using supervised training;
- A clustering model which uses unlabelled data and is trained using unsupervised learning to sort the data into clusters with similar properties based on the input audio-linguistic representations, where this clustering of the data may be used to extract previously unknown health related trends in the input speech data.

There are a number of ways in which task-specific training of the general model structure of Figure 2 may proceed. The task-specific fine-tuning method 200 may comprise training of the pre-trained encoder 205 and the task specific decoder 207 together, e.g. by training the models together to map the input sequence 204 to the target output 208, 209, 210, 211. In this way the encoder 205 may continue to learn refined audio-linguistic representations for the specific task chosen for fine tuning. Alternatively, the pre-trained sequence encoder 205 may be fixed such that it continues to provide the same audio-linguistic representations for a given input sequence 204 and is no longer trained to alter these representations after pre-training. In these embodiments, it is only the task-specific machine learning model (the decoder) 207 which is trained to map the output audio-linguistic representations to the particular target output.

Using the two step training method in this way, large widely available unlabelled speech based training sets can be used to learn multipurpose audio-linguistic representations for clinical applications, before further targeted training on less widely available smaller health related data sets can be used to refine the audio-linguistic representations for a particular health related speech processing task. Further, by being trained initially to be multipurpose, audio-linguistic representations can generalise better on task-specific applications.

It will be appreciated that the above described overview of the training method can be implemented with many different model architectures and training strategies as long as they achieve the broad requirements set out in the above figures. Below are set out certain specific and non-limiting examples of the two training steps, including possible choices for the model architecture and preparation of the input sequence.

### Example 1 - Pre-training using a tokenisation method

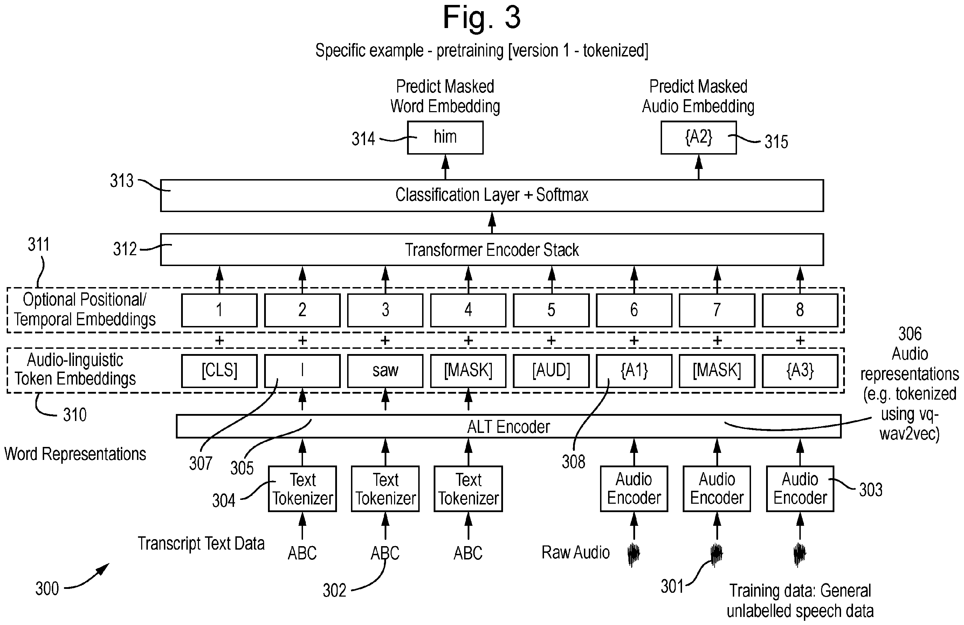

Figure 3 illustrates one specific way in which the pre-training method of Figure 1 may be realised. In this example the main model 312 architecture is based on the Transformer architecture (see "Attention is all you need", Vaswani 2017, arXiv:1706.03762v5). The training scheme comprises mapping an input sequence 310 of text representations 302 and audio representations 308 to a target output 314 and/or 315 such that the Transformer encoder 312 learns combined audio-linguistic representations. The training is based on a denoising objective in which one or more audio 308 or linguistic 307 representations are masked 309, 316 and the model is trained to predict the masked tokens 314, 315. There are a number of optional and preferable features chosen in this specific example, not essential to the overall method and training strategy, but associated with particular advantages as will be explained.

The input of the model is raw audio data 301 containing generic speech data. In this case, text data of the transcript 302 contained within the audio data 301 is also provided, but this may equally be extracted in a separate process directly from the raw audio data using known ASR techniques. The first stage in the model is preparing the combined audio-linguistic input sequence 310. As described above, the linguistic and audio representations may simply be vectors encoding information associated with the text and audio respectively. In these cases, the raw audio 310 is passed through an audio encoder 303 which computes a vector representing a segment of the raw audio data. The audio encoder 303 preferably calculates representations of sub-word level spans of audio, which is important for health application because it allows information present in short time scales of the audio to be captured by the representations. For example, 30ms sliding windows of the raw audio 301 at 10ms steps may be taken and transformed into the audio representations 308. These windows may be variable in length and may or may not overlap with one another.

The output of the audio encoder 303 therefore is a number of audio representations 308 which together represent a time period of the raw audio 301 data. There are many such methods for calculating vectors based on segments of raw audio (e.g. wav2vec; learning deep representations by signals, 2017 IEEE international conference on data mining, Yuan, 2017). Similarly, the linguistic representations 307 are formed by computing vectors, in this case representing individual words within the training data, such that text is fed into the text encoder 304 and the output is vectors representing a word or sub-word piece of the input text data 302. Again, the text encoder may use any known method to calculate any vector representing a word sub-word or multi-word text segment (for example using BERT related methods or word2vec). Although in general any type of word or audio representation may be used, in some embodiments particular performance advantages can be obtained by using text and audio tokens, as will now be illustrated.

In the specific method of Figure 3, the text encoder 304 and audio encoder 303 are configured to provide tokenised representations of the words and audio segments. In particular, the representations may be quantised with the model configured to take as its input a sequence of these audio-linguistic tokens 310 where each token represents a generalised word from a fixed size extended audio-linguistic vocabulary. In this case, the text representations are text tokens which are tokens indicating a word fragment or piece of punctuation such that any sequence of text can be formed by a selected sequence of text tokens from the overall audio-linguistic vocabulary. Similarly, the audio representations are vector quantised such that all audio segments may be represented by an audio token which indicates a vector quantised representation of a sound. In this way, any sequence of speech can be reconstructed into an input sequence of audio-linguistic tokens 310 comprising audio tokens 308 and text tokens 307. Tokenisation can have a number of performance advantages in training the model which assists with the prediction of masked tokens and the efficient learning of combined audio-linguistic representations.

Describing one specific example of tokenisation in more detail, the model takes as input a variable-length sequence of audio-linguistic tokens (ALTs). Here, an ALT is an integer which represents a generalized "word", i.e. a discrete unit, from an extended audio-linguistic vocabulary.

Each generalized word may correspond to one of four things:
- **Text token:** An ALT for a written word or piece of punctuation, e.g. [dog] or [?] . Text tokenization may be performed in a standard way; (see e.g."Attention Is All You Need", Vaswani et al. 2017, arXiv:1706.03762v5).).
- **Audio token:** An ALT for a vector-quantized representation of a sound, e.g. {A1} or {A2} .
- **Corruption token:** An ALT representing one or more missing tokens that have been purposefully dropped for self-supervised training, e.g. <MASK> in Fig 3.
- **Special token:** An ALT used to define the structure of the ALT sequence, e.g. [CLS] or [AUD], to assist the model in interpreting the sequence.

Two ALTs representing the same generalized word are always identical and two representing different generalized words are always distinct. A sequence of ALTs may therefore represent a piece of (possibly corrupted) text, a piece of (possibly corrupted) audio, or a combination of the two. Audio ALTs are created
from a raw sound wave using a data-driven vector quantization method (e.g. vq-wav2vec) on a 30ms sliding window with stride length 10ms; this is trained separately.

ALTs cannot occur in any order and must obey the following rules:
1. The first token must always be the classifier special token [CLS] .
2. Any text tokens (including text corruption sentinels) must immediately follow [CLS].
3. The next token must be the audio special token [AUD], unless there are no audio tokens.
4. Any audio tokens (including audio corruption sentinels) must immediately follow [AUD].

Each audio token typically represents a sub-word-level span of audio; many hundreds of audio tokens typically correspond to one word. We do not perform any kind of word-level alignment. The need to preserve sub-word features such as hesitations and stutters for certain health applications (e.g. diagnosing brain injury, degenerative brain conditions, and affective disorders) motivates this formulation of the audio tokens.

Using this schema, any finite piece of text, audio or a combination thereof can be translated into a finite sequence of ALTs. For the method to work optimally, if audio and text tokens appear in the same sequence, the text tokens should represent a reasonable transcription of the speech in the audio. In this example, the generalized word vocabulary size (including audio tokens, text tokens, sentinels and special tokens) is about 61,000.

The output of the text encoder 304 and audio encoder 303 is therefore a sequence of audio-linguistic tokens 307, 308, 309 which encodes a sequence of the input audio training data 301. The model may then comprise an audio-linguistic token embedding layer 310 which learns during training of the model to map the input audio-linguistic tokens (ALTs) to a vector space which encodes contextual information regarding the tokens and their position within the sequence. There is also optionally a sequence position embedding layer 311 which learns to map the sequential numbering of the audio-linguistic tokens to a sequence position representation. To assist with temporal alignment in the case of unaligned audio and linguistic sequences, this sequence position embeddings can additionally contain start and stop time information for each element in the sequence. The embeddings (or equivalently representations) of the sequential numbering and the corresponding audio-linguistic token embedding are then summed to form the input sequence of representations which are fed to the Transformer encoder stack 312.

In this case, words and representations are aligned so that the network can map between them. Alternatively, real time information within the input audio wave could be encoded such that no alignment is necessary. In the specific example of Figure 3 the model comprises a Transformer encoder stack 312 and a classification layer and soft max 313, the Transformer encoder stack maps the input representations to combined audio-linguistic representations which are output to the classification and soft max layers. The classification and soft max layers map the output audio-linguistic representations to a probability distribution, with the highest probability selected to determine the predicted masked token 314, 315.

The training process comprises providing speech data in the form of more audio together with text data (where the text data is optionally derived from the raw audio on a separate process). The text encoders and audio encoders are fixed and simply translate the raw audio to the audio representations or more specifically in this example, to the ALTs. The audio-linguistic token embedding layer 310 the positional embedding layer 311 the Transformer encoder stack 312 and the classification layer and soft max 313 are trained together using self supervised learning to predict masked words and/or acoustic tokens, exploiting the interdependence of the text and audio to learn strong combined audio-linguistic representations containing rich information on the input speech data. The output of the Transformer encoder stack is therefore 8 vectors which are the audio-linguistic representations which are then classified by the classification layer to predict the masked tokens 314, 315.

After training, the added layers, the classification layer and softmax, are discarded and the pre-trained Transformer encoder 312 can then be used to convert any input sequence of audio representations and text representations into the output audio-linguistic representations for a downstream task. The pre-trained encoder can then be applied to any health related task in which speech analysis can be used to extract deep information to identify, monitor or evaluate a health condition. However, to optimise the learned audio-linguistic representations for a specific speech processing task, fine tuning can be carried out in the model of Figure 3 to achieve the required specific optimisation.

### Example 1 - Fine tuning using a tokenisation method

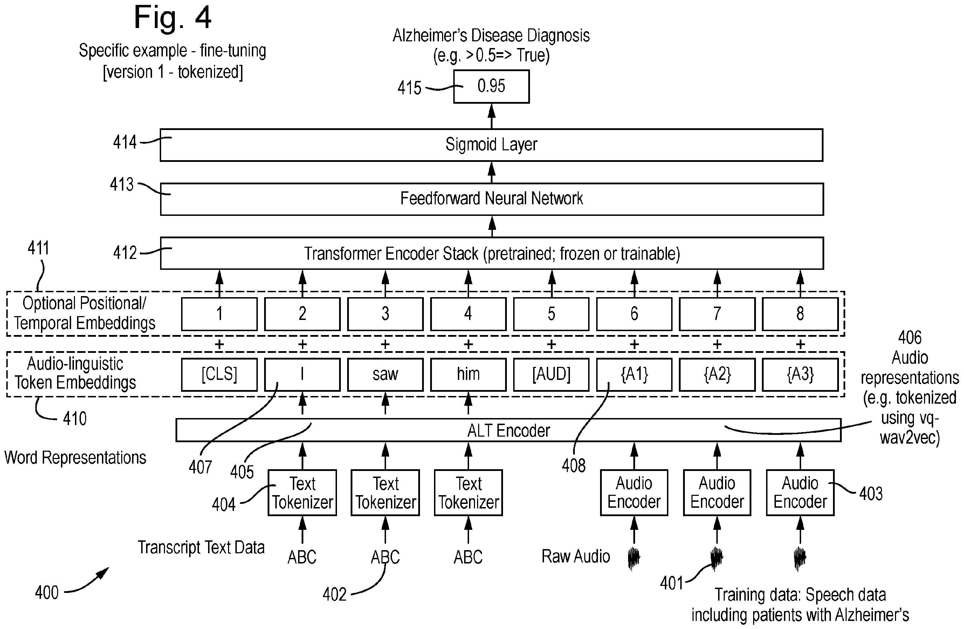

Figure 4 schematically illustrates a specific example of the fine-tuning of the pre-trained model shown in Figure 3. The fine-tuning method utilises the pre-trained Transformer encoder stack 412, pre-trained according to the method of Figure 3.

In this case, again the input data is raw audio speech data but here the speech data is clinically relevant speech data, i.e. speech data including speech from patients with a particular health condition. The particular example of Figure 4 illustrates a method of fine-tuning the model to learn combined audio-linguistic representations for diagnosing patients with Alzheimer's disease. As described with respect to Figure 3, in this example the raw speech audio is prepared as a sequence of audio-linguistic tokens 410 representing a sequence of the speech data. Although in other examples the representations need not be quantised this method illustrates the use of vector quantised tokens to represent the speech data.

As described above, the input raw audio speech and text transcript of the speech is prepared as the audio-linguistic token sequence 410. The method preferably includes using an audio-linguistic embedding layer for mapping the tokens to a contextual representation encoding information about their position in the sequence and other contextually relevant information. The model also includes the optional positional sequence embedding layer 411 which encodes the position of a particular token in the sequence in a vector space representation.

The vectors output by the audio-linguistic token and embedding layer and the positional embedding layer are summed and fed to the pre-trained Transformer encoder stack 412. The pre-trained encoder stack 412 is appended with one or more additional network layers 413, 414 suitable for performing a specific task, in this case Alzheimer's diagnosis. In this case, the network layers are a feed forward near on network 413 and a sigmoid layer 414 which together map the sequence of output audio-linguistic representations to a target value 415 representing an Alzheimer's disease diagnosis (e.g. where greater than 0.5 corresponds to a positive diagnosis).

Using labelled training data where the labels indicate a known Alzheimer's diagnosis the model can be trained to map the audio-linguistic representations to the diagnosis value 415. There are a number of different ways in which this can be achieved. Firstly, the trained Transformer encoder stack could 412 simply be frozen i.e. not part of the ongoing task specific training but simply left in its state following pre-training in which it translates a sequence of audio-linguistic tokens to a combined audio-linguistic representation sequence which can be classified to provide the diagnosis. In this case, it is just the feed forward neural network 413 and sigmoid layer 414 which are trained to map the output audio-linguistic representations from the Transformer encoder stack 412 to the target diagnosis value 415. Alternatively, the model of Figure 4 (excluding the fixed text encoder 404 and audio encoder 403) can be trained end-to-end such that the Transformer encoder stack 412 learns refined audio-linguistic representations specifically for the present task, in this case the diagnosis of Alzheimer's. This provides particular advantages in that further optimised body linguistic representations are learned by the Transformer encoder stack which are particularly discriminative of the health condition, such that the model can later be applied on unlabelled patient data to provide a diagnosis of improved accuracy.

Following the fine-tuning step illustrated in Figure 4, the model can then be applied in a clinical context to diagnose Alzheimer's. In this situation, speech data is taken from a patient and prepared as a sequence of audio-linguistic tokens as explained above. This input sequence is fed to the pre-trained and fine-tuned Transformer encoder which outputs audio-linguistic representations of the patient's speech which are particularly well formulated for the diagnosis of Alzheimer's. The trained neural network and sigmoid layer then convert these to a diagnosis value 415 associated with the patients likelihood of having Alzheimer's. As explained throughout, it is the extraction of the relational interdependence of the words used by the patient and the acoustic information related to the way the words are enunciate which provides which information allowing an accurate diagnosis of a multitude of health conditions.

### Example 2 - Pre-training example using temporal attention

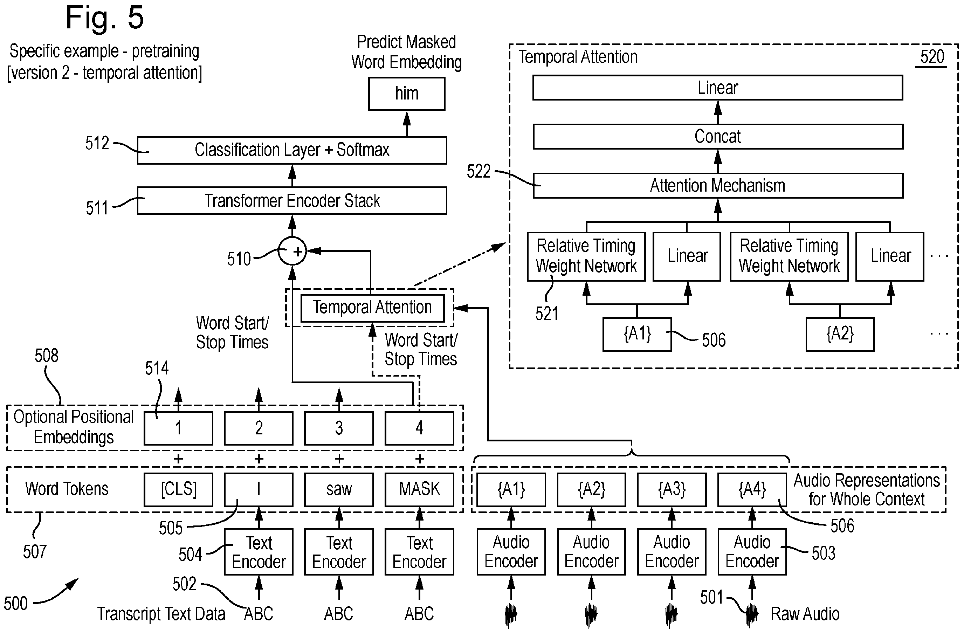

Figure 5 schematically illustrates a further example of a method of pre-training a machine learning model for performing a speech analysis task to monitor or diagnose a health condition. This method utilises the same core principal of obtaining linguistic representations and audio representations of a sequence of speech data and using self-supervised learning to map the sequence to a target output to learn combined audio-linguistic representations which encode the interdependence between words and acoustic information in speech (as well as independent acoustic and linguistic information which is derivable from the audio and linguistic representations individually) which has particular clinical significance and be used for a wide range of health related speech processing tasks.

The temporal attention method 500 of Figure 5 shares the core common features of the tokenisation method of Figure 3 including first computing a number of linguistic representations 505 each encoding a word or subword (or in some cases multi word sequence) within a sequence of the speech data 501 and computing a number of audio representations encoding the audio content of segments of the raw audio data. However, the temporal attention method of Figure 5 differs in the way the representations 505, 506 are combined into the input sequence of audio and linguistic representations which are then mapped to the combined audio-linguistic representations.

In this case, the word tokens (although these could remain linguistic representations in other examples) are passed to an embedding layer 507 to compute word token contextual embeddings. As before, an optional sequence position embedding layer 508 computes a sequence position representation encoding contextual information of the sequence position. Where the sequence position layer is included, the sequence position embeddings are summed with the corresponding word token embeddings 505 and the resulting linguistic representation sequence fed to a first layer of the encoder stack (in this specific example a Transformer encoder, as described above).

The sequence of audio representations 506, together encoding segments of the audio data which include the encoded words, is passed through a the temporal attention layer (TAL) that learns to select and combine the relevant audio representations based on the time differences between the start/end of the word in question and the start/end of the audio representations. The result is a sequence of audio embeddings of the same length as the sequence of linguistic embeddings, which is then summed together with the linguistic embeddings early in the model 510, 610. The resulting sequence is then passed to the Transformer encoder stack. (for a detailed description of the implementation of a related architecture, see for example "Self-Attention with Relative Position Representations", Shaw et al. 2018, arXiv:1803.02155v2).

The TAL 520 makes use of the relative timing information of the audio representations, e.g. positive/negative time difference between the start/end of the word and the middle of the audio representation, which is fed to a relative timing weight network 521 within the TAL 520, as shown in the enlarged schematic of the TAL 520 in Figure 5. This creates the weight that is used in the attention mechanism.

This structure allows the network to "attend over" (i.e. be aware of) the whole audio sequence at each word step and use the relative timings to pull in audio information where it's relevant. The word representation 505 in question may pull in audio (in particular prosodic) information from a very different time, e.g. a rising inflection at the end of an utterance interacts with words around the start of the utterance which are then more likely to be question words.

After the TAL layer 520 the audio representations and linguistic representations are added at the addition layer 510 to form a combined sequence of audio 506 and linguistic 505 representations. The temporal addition layer 520 and the addition layer 510 therefore provide an alternative method to that of Figure 3 of forming the input sequence of audio and linguistic representations which is then mapped by the model (i.e. the remaining Transformer encoder stack 511 and classification layer and softmax layer 512) to the target output 513. The initial layers 520, 510 can therefore be considered an alternative means to the multiple embedding layers of Figure 3 of forming the "input sequence".

As with all pre-training implementations, the model may be trained on large unlabelled data sets using self-supervised learning in order to learn the audio-linguistic representations. In this case, the linguistic representations may be corrupted (i.e. by masking a text token in this example) and the model trained to predict the correct word token. The output of the final Transformer encoder stack 511 is a sequence of combined audio-linguistic representations which are then fed to the classifier and softmax which determine a probability of each token and select that with the highest probability. This method enjoys the same advantages of the tokenisation pre-training method (and all pre-training methods of the current invention) in that the model can be trained using large unlabelled and widely available datasets using self-supervised learning to learn audio-linguistic representations encoding clinically relevant speech information for a downstream speech analysis task for monitoring or diagnosing a health condition.

### Example 2 - Fine-tuning using temporal attention

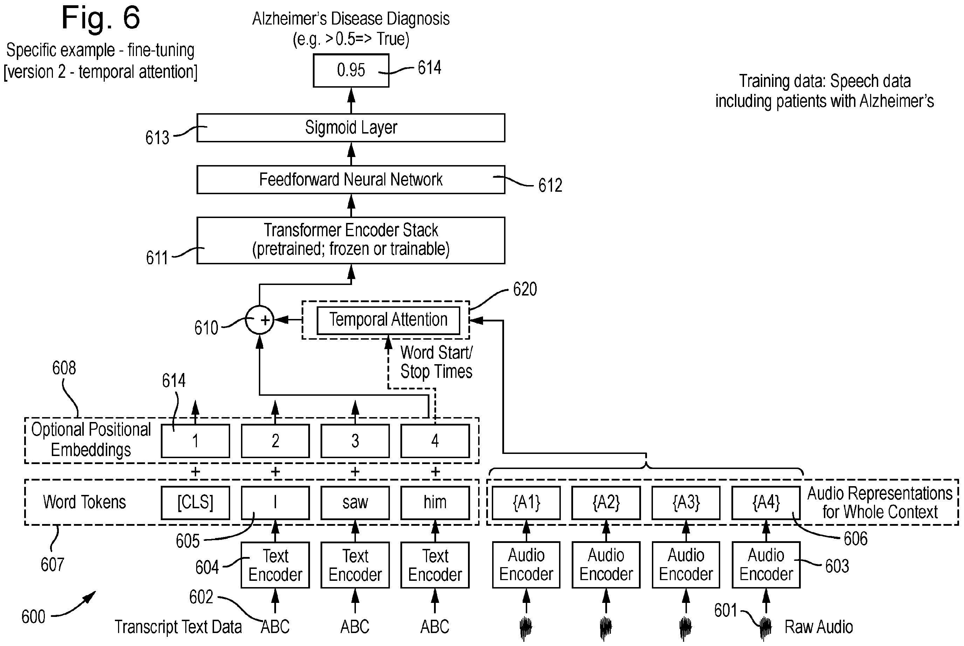

Figure 6 schematically illustrates additional method steps for fine-tuning the model-trained according to the method of Figure 5 for a specific speech analysis task for the prediction, monitoring, diagnosis or evaluation of a health condition. The example of Alzheimer's disease diagnosis is selected to illustrate the method, but the method could be applied to any health condition, and to other task objectives (e.g. monitoring disease severity over time).

As with all variants of the fine-tuning method according to the present invention, the guiding principle is that once the encoder has been trained to map input speech data to combined audio-linguistic representations which encode information on the interaction between spoken words and acoustic (e.g. prosodic) information, these representations can be used for a specific speech analysis task, either directly by appending an additional network layer and training the layer to map the combined audio-linguistic representations to a target output associated with a health condition, or by training the pre-trained encoder together with the additional network layers such that in the process of training further refined audio-linguistic representations are learned for that specific speech analysis objective. In either case, the result is a trained model that can be applied to patient speech data to extract meaningful information about the patient's speech.

Returning to the example of Figure 6, as described above in relation to Figure 4, the training data here is speech data associated with a particular health condition. For example, it may be collected speech from a group of people where it is known some of them have a particular condition. In the specific example of Figure 6 the training data relates to Alzheimer's.

As before, the first step is to covert the input speech data into linguistic representations encode words or subwords and audio representations encoding audio information from temporal segments of the audio data. Preferably, as with all embodiments of the invention, the audio encoder may be trained to extract non-linguistic audio content, as is explained in more details below. However the method can equally work on generic audio representations.

In this example the text encoder transforms the input text data to word tokens indicating a word or subword from a fixed size vocabulary which can improve aspects of the training but is not strictly required. As above the word tokens are encoded into the word token embeddings 605 which are combined with sequence position emeddings encoding contextual information of the sequence positions. The linguistic representations 614 and audio representations 606 are combined after the first Transformer encoder stack layer 609 by the temporal attention layer 620 and this input sequence of audio representations and linguistic representations, encoding a segment of the speech, is input into the pre-trained Transformer encoder. The output of the final stack of the pretrained Transformer encoder 611 is a sequence of audio-linguistic representations which encode relational information capturing the interaction of linguistics and prosody.

As explained above, any appropriate network layers can be selected appended to the pre-trained encoder to perform any speech processing task, for example classification, regression, clustering or sequence-to-sequence transformation. In this example, a feedforward neural network 612 and sigmoid layer 613 are appended for training to map the audio-linguistic representations to a target value 614 associated with a prediction of an Alzheimer's diagnosis. In particular, by using training data labelled according to a known Alzheimer's diagnosis the appended network layers can be trained to recognise signals in the audio-linguistic representations indicative of the presence of Alzheimer's.

Again, there are two possibilities for the fine-tuning training scheme. Firstly, the pre-trained Transformer encoder may be fixed (i.e. so that it reproducibly maps speech to the same audio-linguistic representations) and the appended task-specific network layers 612, 613 may trained alone to learn how to identify the Alzheimer's patients from the audio-linguistic representations. Alternatively, and preferably for some tasks, the Transformer encoder stack may continue to be trained together with the task-specific network layers so that they learn joint optimised configurations which may provide optimised speech processing for the particular task and accordingly, in this case, more accurate diagnosis of Alzheimer's. In either case, once the model has been fine-tuned on labelled data the model can be applied to unlabelled patient speech data to provide a reliable diagnosis of Alzheimer's.

### Using prosodic representations

The fundamental principle of the invention is the learning of combined audio-linguistic representations, encoding clinically relevant interdependent speech-audio information, for speech processing tasks. As illustrated above, an audio encoder is used to map segments of input audio speech data to audio representations which are then used in the input sequence for training. However, for many applications of the invention, speech processing tasks can be further optimised by being selective about the audio information encoded in the audio representations. One particularly advantageous example is encoding the prosodic information contained in a segment of the audio data.

The prosodic information, as defined herein, encompasses all variations in the input audio signal except the variations determined by the actual words used in the speech, i.e. "normative phonetic information". The prosodic information encompasses changes in the input audio accounted for by speaker identity, traits (gender, age, accent), and states (e.g. emotionality), intonation/inflections and the acoustic environment. It also captures errors in speech (other than errors relating to choice of words) for example due to mispronunciation and errors in correctly forming the chosen words, i.e. phonetic errors and deviations.

The inventors have identified that by training prosodic-linguistic representations, rather than more general audio-linguistic representations, even richer and more discriminative information can be encoded to allow for enhanced speech processing for specific applications, particularly for monitoring and diagnosis of specific health conditions.

The above described methods of pre-training and fine-tuning apply equally to when prosodic representations are used in place of general audio representations. The only difference is that in place of the general audio encoder illustrated in the above figures a prosody encoder is used which is specifically trained to output prosodic representations of input segments of audio data which encode the non-linguistic variations in the raw audio data. These prosodic representations can simply be used in place of the generic audio representations throughout the above described methods to achieve the additional advantages in terms of more accurate speech monitoring and diagnosis. The following outlines a description of a method of training a prosody encoder to be used in place of the audio encoder of the above methods.

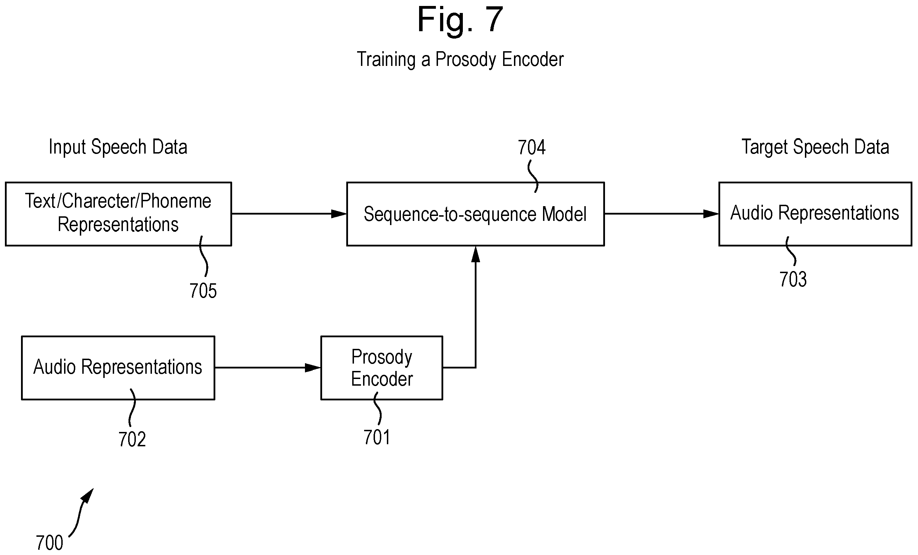

Figure 7 schematically illustrates an overview of a general training method 700 for training a prosody encoder 701. Input audio representations 702 of input speech data and mapped to the same audio representations as an output 703 through an information bottleneck associated with the prosody encoder 701 so that the prosody encoder is forced to learn reduced dimension representations of the audio representations. By conditioning the prosody encoder 701 by providing information on the text, i.e. linguistic, content 705 of the input speech data, the prosody encoder is trained to exclude the linguistic content from the reduced dimension representations in an effort to more accurately reconstruct the target audio representations. The prosody encoder 701 therefore learns representations which encode the non-linguistic content of input audio segments. The trained prosody encoder is then used in the pre-training and fine-tuning methods of the present invention in exactly the same way as the general audio encoder described above.

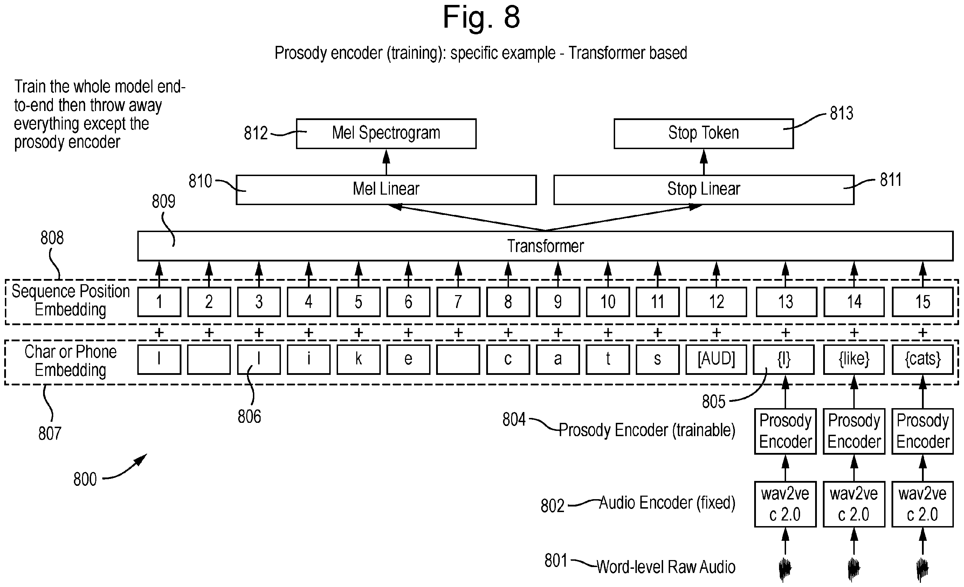

A specific example of a method of training a prosody encoder 704 is schematically illustrated in Figure 8 which follows the general principle described above. The raw audio 801 is firstly converted to audio representations, in this specific example at the word level using a fixed audio encoder 802 (for example using "wav2vec 2.0: A Framework for Self-Supervised Learning of Speech Representations", Alexei Baevski, 2020, arXiv:2006.11477v1). The word-level audio representations are fed to the trainable prosody encoder 804. The model uses a Transformer structure (see for example Neural Speech Synthesis with Transformer Network, Li et al, 2019 arXiv: 1809.08895v3) and maps sequences of character or phoneme 806 representations, together with the audio representations 805 from the trainable prosody encoder 804 to the same input audio 812 (in this case represented in the form of mel spectrogram) via an autoregressive decoder.

As before, since the Transformer is provided with the character/phoneme information present in each of the audio words, the prosody encoder learns to encode representations which encode the variations in the input audio 801 which are not derived from the characters and therefore the words themselves. Instead, the prosody encoder learns to encode all other acoustic information, i.e. prosody as defined herein, in the output prosodic representations. Once trained, the prosody encoder is extracted and used fixed in the methods illustrated above as an instance of the audio encoder. As explained above, learning combined prosodic-linguistic representations allows for information related to the interdependence between words and prosody to be utilised which is highly discriminative for a large number of speech processing tasks, particularly for monitoring and diagnosing health conditions.

A further specific implementation of a prosody encoder which could be used is described in "Towards End-to-End Prosody Transfer for Expressive Speech Synthesis with Tacotron", RJ Skerry-Ryan et. al 2018, arXiv:1803.09047v1. Another alternative is the non-semantic encoder as trained in "Towards Learning a Universal Non-Semantic Representation of Speech", Shor et al 2020, arXiv:2002.12764v5.

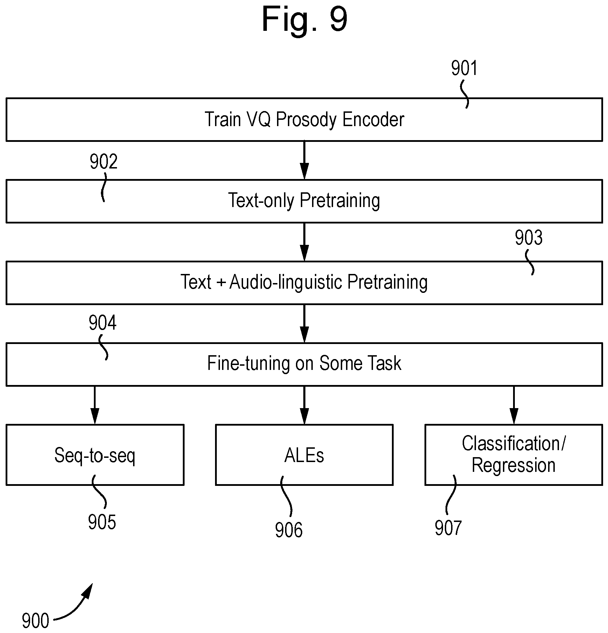

Figure 9 illustrates an example of an overall training sequence for methods utilising a prosody encoder (for other examples, utilising a standard acoustic encoder, the first step can be disregarded). In the following overview, the method is illustrated in the case that the text and audio representations are tokenised but the following is equally applicable to methods in which only the text is tokenised or neither are tokenised and representations are used directly.

Where a prosody encoder is used, the first step 901 is training the prosody encoder, for example using one of the methods described above, such that the prosody encoder is trained to map input audio data sequences to a prosodic representations encoding non-linguistic data. The prosody encoder is then fixed and used in the following training steps.

In step 902, text-only pre-training is carried out. In particular, the machine learning model (for example as shown in Figure 1, 3 or 5) is trained on a large text only corpus. Training is preferably self-supervised using a de-noising objective by randomly corrupting text tokens, for example by replacing them with a corruption token, at a given probability rate, for example 15%. Sequential corruption tokens are consolidated into a single corruption token. The network is trained to 'denoise' the input (i.e. predict the tokens replaced with corruption tokens) based on the textual context. This allows initial training of the model on widely available large text-only datasets.

In step 902, text + audio-linguistic pretraining is carried out, as illustrated in any of Figures 1, 3 and 5. The model is pre-trained on a combination of text-only and audio-linguistic examples, for example sampling from each corpus with 50% probability. We randomly corrupt text tokens (i.e. replace them with a corruption token) with a given probability (e.g. 15%) for text-only examples and a second given probability for text and audio data (where the second given probability may be different, e.g. 25%. Audio tokens may be corrupted with a third given probability (which may be different to the others, for example 8%). Spans of 10 audio tokens may be corrupted together rather than singly. The network learns to 'denoise' the input - based on the context and using the acoustic (audio/prosodic) data to help denoise the linguistic data and vice versa.

The text and audio-linguistic pre-training may optionally include multi-task pretraining in which the network is trained on a combination of text-only and audio-linguistic examples, sampling from each corpus with 50% probability. In this case, text tokens are randomly corrupted (i.e. replace them with a corruption token) with a given probability for text-only examples. For audio-linguistic examples, we do one of three things with equal probability: a. Corrupt all the audio tokens. b. Corrupt all the text tokens. c. Corrupt the text tokens with 25% probability; corrupt the audio tokens with a probability of 8% in spans of 10 token (as per the text + audio-linguistic pretraining, where all probability values are purely exemplary). In each case, the network learns to 'denoise' the input - based on the context and potentially using the acoustic data to help denoise the linguistic data and vice versa.

### Application of the trained model

Once the model has been pre-trained and fine-tuned using task-specific training, it can be used for a particular speech processing task. In particular, when trained for a specific speech processing task for monitoring, diagnosing or otherwise evaluating a health condition, it can then be applied, for example in a medical device. For example the trained model may be stored in a memory or a computing device, either localised or distributed, and speech data may be fed to the model for analysis to determine an indication of a health condition.

A method of using the trained model simply involves feeding in patient speech data to the model trained as in any previously described example to determine a medical condition. Taking the general overview of Figure 2 as an example, patient speech data may firstly be prepared as the required input sequence 204 of linguistic representations 202 and audio representations. The input sequence may then be fed through the model to classify the speech data, perform regression, provide a response or perform clustering to analyse it, though any of the methods described above.

The trained model may therefore be incorporated into a medical device. The medical device may be a fully software based device, for example an API. In other examples it may be incorporated into hardware. The medical device may comprise a memory holding the trained model, an input (for example a microphone) for accepting patient speech data and feeding this to the model and an output for providing the output of the model, for example a display.

There are a huge number of health conditions which leave signals within speech which can be identified by the methods of the present invention. A few limited examples include: where the health condition is related to the brain, e.g. a cognitive or neurodegenerative disease (example: Dementias, Alzheimer's Disease, Mild Cognitive Impairment, Vascular Dementia, Dementia with Lewy Bodies, Aphasias, Frontotemporal Dementias, Huntington's Disease); motor disorders (example: Parkinson's Disease, Progressive Supranuclear Palsy, Multiple System's Atrophy, Spinal Muscular Atrophy, Motor Neuron Disease, Multiple Sclerosis, Essential Tremor); affective disorders (example: Depression, Major Depressive Disorder, Treatment Resistant Depression, Hypomania, Bipolar Disorder, Anxiety, Schizophrenia and schizoaffective conditions, PTSD); neurobehavioural conditions (example: spectrum disorders, Attention-Deficit Hyperactivity Disorder, Obsessive Compulsive Disorder, Autism Spectrum Disorder, Anorexia, Bulimia), head injury or stroke (example: stroke, aphasic stroke, concussion, traumatic brain injury); pain (example: pain, quality of life).

Further limited examples include where the health condition is related to the respiratory system (example: SARS-CoV-2, Whooping cough, Asthma, COPD, Pneumonia, Wet/dry cough, Flu, Common cold, Lower respiratory Infections; Trachea, Bronchus, and Lung cancers; Tuberculosis).

The methods described herein can also be applied to cases where there are multiple different health conditions or symptoms of different health conditions or where the health conditions are not yet known.

The methods described herein can also be applied for the purpose of
- Pre-screening of patients.
- Diagnosis.
- Providing disease-relevant information.
- Symptom monitoring.
- Progression monitoring.
- Therapy-response.
- Therapy-selection.
- Therapy adherence.
- Therapy.
- Stratification.
- Differential diagnosis.
- Assessing comorbidities.
- Disease management

Where labelled data is used for the fine-tuning step the labelling of the data can be a measure derived from one of the following states or traits:
- Physical state (example: gait patterns)
- Physiological state (example: fMRI, amyloid beta levels in the brain)
- Psychological state (example: emotional state).
- States that can be physiological and/or psychological (example: stress, fatigue).
- Pathological condition.
- Cognitive state (example: working memory impairments).
- The speech production process itself (example: aphasias).
- Diagnosis or probable diagnosis (example: mild cognitive impairment)
- Symptoms of a health condition (example: cognitive decline (Alzheimer's), motor impairment (Parkinson's), high negative affect (Depression)).
- Established scales or diagnostic methods that have been associated with a health condition (e.g. the Clinical Dementia Rating Scale, amyloid imaging (Alzheimer's); the MDS-Unified Parkinson's Disease Rating Scale, DaT-SPECT imaging (Parkinson's); Mini International Neuropsychiatric Interview (Depression).
- Biomarkers that have been associated with a health condition (e.g. Neurofilament Light Chain (Alzheimer's, Huntington's).
- Parameters obtained through wearables, smartphones or other digital technology.
- Behavioural patterns.
Alternatively, where labelled data is used for the fine-tuning the labelling of the data are different from these states and traits above, but contains information that can improve the assessment of those states or traits (e.g. age, gender); examples of how assessment can be improved include increasing generalisability, robustness, reliability, safety, and/or explainability.

### Alternative embodiments and implementations

It will be appreciated that the above description outlines only certain exemplary embodiments of the methods according to the present invention and there are many alternative embodiments and examples that fall within the scope of the present concept.

One particularly important alternative is that the rich speech data extracted by the methods of the current invention, need not necessarily be applied to purely health-related applications but this rich information can be used more widely in other speech processing tasks. Certain limited examples include:
- Automatic speech recognition.
- Diarisation (separating speakers during automatic speech recognition).
- Lie detection.
- Sarcasm detection.
- Personality prediction.
- Sentence acceptability.
- Sentiment analysis.
- Paraphrasing/sentence similarity.
- Natural language inference.
- Coreference resolution.
- Sentence completion.
- Word sense disambiguation.
- Question answering.
- Machine translation.
- Understanding intent.
- Conversational agents such as chatbots.

In these cases, the model is pre-trained as explained above, for example using the methods described with reference to Figures 1, 3 and 5. Then, instead of using a health related task-specific training step, as shown in Figures 2, 4 and 6, a task-specific training step relating to the required speech processing task (for example one of the above) is used instead. The method proceeds in exactly the same way and requires no alteration, other than the objective chosen for the fine tuning (task specific training). For example the model can be trained using a classification layer for sentiment analysis and trained on speech data labelled according to sentiment and the model trained to classify input speech, using the additional information present in the interrelation between words used and the audio (preferably prosodic information) to accurately classify segments of speech according to sentiment.

## Claims

1. A computer-implemented method of training a machine learning model for performing speech analysis for monitoring or diagnosis of a health condition, the method using training data comprising audio speech data, the method comprising:
obtaining one or more linguistic representations that each encode a sub-word, word, or multiple word sequence, of the audio speech data;
obtaining one or more audio representations that each encode audio content of a segment of the audio speech data;
combining the linguistic representations and audio representations into an input sequence comprising:
linguistic representations of a sequence of one or more words or sub-words of the audio speech data; and
audio representations of segments of the audio speech data, where the segments together contain the sequence of the one or more words or sub-words; the method further comprising:
training a machine learning model using unsupervised learning to map the input sequence to a target output to learn combined audio-linguistic representations of the audio speech data for use in speech analysis for monitoring or diagnosis of a health condition.

2. The method of claim 1 wherein training the machine learning model comprises training using self-supervised learning.

3. The method of claim 2 wherein training the machine learning model comprises masking or corrupting one or more of the linguistic and/or audio representations in an input sequence and training the machine learning model to predict the masked or corrupted linguistic and/or audio representations.

4. The method of any preceding claim wherein combining the linguistic representations and audio representations comprises:
forming a linguistic sequence comprising linguistic representations of a sequence of one or more words or sub-words of the audio speech data;
forming an audio sequence comprising audio representations of segments of the audio speech data, where the segments together contain the sequence of the one or more words or sub-words; and
combining the linguistic sequence and audio sequence by one or more of:
concatenating the linguistic sequence and audio sequence along any dimension;
summing the linguistic sequence and audio sequence;
performing a linear or non-linear transformation on one or both of the audio sequence and linguistic sequence
combining the linguistic sequence and audio sequence by inputting to an initial neural network layer.

5. The method of claim 4 wherein combining the linguistic sequence and audio sequence comprises:
training a neural network layer to align the audio sequence with the linguistic sequence by, for each linguistic representation, selecting one or more relevant audio representations using temporal alignment information, where the model obtains the temporal alignment information from the audio sequence by determining the time delays between the linguistic representation and each audio representation.

6. The method of any preceding claims further comprising:
pre-training the machine learning model using unsupervised learning on a first training data set to map the input sequence to a target output to learn combined audio-linguistic representations of the audio speech data;
adding a task-specific network layer after the machine learning model and performing task-specific training using a second training data set comprising task-specific training data, associated with a specific health monitoring or diagnosis task.

7. The method of claim 6 wherein performing task-specific training comprises:
training the pre-trained machine learning model and the task-specific layer together to map an input sequence to a target output associated with a health condition.

8. The method of any preceding claim wherein the health condition is related to one or more of a cognitive or neurodegenerative disease, motor disorder, affective disorder, neurobehavioral condition, head injury or stroke.

9. The method of any preceding claim wherein the linguistic representations each encode a character or phoneme of the audio speech data.

10. The method of any preceding claim wherein the audio representations comprise prosodic representations that each encode non-linguistic content of a segment of the audio speech data.

11. The method of claim 10 wherein obtaining a prosodic representation comprises inputting a segment of audio data into a prosody encoder trained to map an audio speech data segment to a prosodic representation encoding non-linguistic content of the audio speech data segment; wherein the prosody encoder is trained by:
training a sequence-to-sequence autoencoder comprising an encoder for mapping input audio data to a reduced dimension representation and a decoder for reconstructing the input audio data from the reduced dimension representation;
conditioning the autoencoder by providing information on the linguistic content of the audio data during training such that the autoencoder learns representations which encode the non-linguistic content of the input audio data;
using the trained encoder of the autoencoder as the prosody encoder.

12. The method of any preceding claim wherein each linguistic representation comprises a text token indicating a subword, word or multi-word sequence from a fixed-size unified vocabulary and wherein each audio representation comprises an audio token indicating a vector quantized audio representation encoding the audio content of a segment of audio data containing one or more words or subwords; wherein together the text tokens and audio tokens form a fixed-size audio-linguistic vocabulary, such that any input segment of audio speech data can be represented by a sequence of text tokens and audio tokens.

13. A computer-implemented method of using a machine learning model for performing speech analysis for monitoring or diagnosis of a health condition, the method using user data comprising audio speech data taskthe method comprising:
obtaining one or more linguistic representations that each encode a word or sub-word of the audio speech data;
obtaining one or more audio representations that each encode audio content of a segment of the audio speech data;
combining the linguistic representations and audio representations into an input sequence comprising:
linguistic representations of a sequence of one or more words or sub-words of the audio speech data; and
audio representations of segments of the audio speech data, where the segments together contain the sequence of the one or more words or sub-words; the method further comprising:
inputting the input sequence of into a machine learning model trained according to the method of any of claims 1 to 12 to provide an output associated with a health monitoring or diagnosis task.

14. A system for performing a speech processing task comprising data processing means configured to perform the method of claim 13.

15. A system for training a machine learning model comprising data processing means configured to perform the method of any of claims 1 to 12.
